# EUROPEAN PATENT SPECIFICATION

(11) **EP 4 121 000 B1**
(45) Date of publication and mention of the grant of the patent: **29.07.2026**
(21) Application number: 21711594.8
(22) Date of filing: 17.03.2021
(51) Int. Cl.: A61K 9/00, A61K 9/08, A61K 38/19, A61K 47/10, A61K 47/26, A61K 47/42, A61P 11/00, A61P 31/04, A61P 11/04

(54) **LIQUID FORMULATION OF GM-CSF FOR INHALATION**
FLÜSSIGE FORMULIERUNG VON GM-CSF ZUR INHALATION
FORMULATION LIQUIDE DE GM-CSF POUR INHALATION

(30) Priority: 17.03.2020 US 202062990810 P; 20.03.2020 EP 20164648
(43) Date of publication of application: 25.01.2023
(73) Proprietor: Drugrecure ApS, 2970 Hørsholm (DK)
(72) Inventor: SKRIVER, Lars, 2970 Hørsholm (DK); WATTS, Alan, 2970 Hørsholm (DK)
(74) Representative: Høiberg P/S
(86) International application number: PCT/EP2021/056851
(87) International publication number: WO 2021/185921

(56) References cited:
- EP-A1- 1 941 901
- WO-A1-2007/009208
- WO-A1-2012/021088
- WO-A1-2020/002650
- CN-A- 1 739 793

## Description

### Technical field

The present invention relates to a liquid formulation comprising recombinant human granulocyte macrophage colony stimulating factor (rhGM-CSF) for inhalation.

### Background

Granulocyte-macrophage colony stimulating factor, GM-CSF, was originally identified as a hemopoietic growth factor. Human GM-CSF stimulates the growth of myeloid and erythroid progenitors in vitro and activates monocytes, macrophages and granulocytes in several immune and inflammatory processes (Gasson JC et al. Prog Clin Biol Res. 1990;352:375-384.; Gasson JC et al. Prog Clin Biol Res. 1990;338:27-41; Hart SP et al. Biochem Soc Trans. 1998;26(4):650-652; Rapoport AP et al. Blood Rev. 1992;6(1):43-57.). It is produced by a number of cell types including lymphocytes, monocytes, endothelial cells, fibroblasts and some malignant cells (Metcalf D. Blood. 1986;67(2):257-267; ; Clark SC, Kamen R. Science. 1987;236(4806):1229-1237; Hart PH et al. J Immunol. 1988;141(5):1516-1521; Metcalf D et al. Blood. 1986;67(1):37-45). In addition to having a function of growth stimulation and differentiation on hemopoetic precursor cells, GM-CSF also was discovered as having a variety of effects on cells of the immune system expressing the GM-CSF receptor (for review see: Hamilton JA. Trends Immunol. 2002;23(8):403-408; de Groot RP et al. Cell Signal. 1998;10(9):619-628).

Granulocyte-macrophage colony stimulating factor inhalation therapy has been disclosed for treating patients suffering from autoimmune pulmonary alveolar proteinosis (aPAP) (Tazawa R et al. N Engl J Med. 2019;381(10):923-932) non-tuberculous mycobacterial (NTM) infection (Scott JP et al. Eur Respir J. 2018;51(4):1702127), acute radiation syndrome (Singh VK et al. Cytokine. 2015;71(1):22-37), acute respiratory distress syndrome (Herold S et al. Am J Respir Crit Care Med. 2014;189(5):609-611) and cystic fibrosis (Heslet L et al. J Inflamm Res. 2012;5:19-27. ) by nebulization.

Inhalation therapy offers the best potential for optimal delivery to the lungs with reduction in systemic side effects. Inhalation treatment targets the airways, lowering the dose of the drug required for optimal efficacy. Also, a low incidence of systemic side effects normally occurs with a rapid onset of drug action. Nebulizers are a very suitable method of treatment for lung infestations of fungi or bacteria as they fill the air with a very fine mist of respirable droplets capable of depositing in all regions of the lung.

However, protein formulation as a liquid for inhalation therapy is a highly complex process as it requires the product to be ready for addition to a nebulizer and remain stable during aerosol generation. The formulation must maintain stability upon long term storage, minimize the effect of nebulization on the integrity of the protein and have appropriate tonicity for pulmonary delivery. Thus, a fine balance between stability, tolerability in the lungs, including potential toxicity, and isotonicity is key to achieve an efficient formulation of protein for inhalation therapy.

The same challenges do not arise when proteins are formulated as a lyophilized product for resuspension, as isotonicity can be achieved upon resuspension and long term stability is ensured only for the dried lyophilate. Thus, the same excipient composition is not always applicable for two different formulations

A liquid formulation of GM-CSF (LEUKINE^{®}) has previously been disclosed for i.v. administration (US2002141970A1). US2002141970A1 suggest addition of a chelating agent as an excipient to increase stability of the compound.

The formulation of drug solution for nebulization is designed to optimize drug solubility and stability; small changes in formulation may also affect inhaled mass, particle size distribution, and treatment time.

Nebulization carries an inherent risk of destabilizing proteins. The main reason for protein instability upon nebulization is unfolding and aggregation at the air-liquid interface, and thus the use of GM-CSF for nebulization requires a stable liquid formulation, which is ready for use for inhalation and does not result in significant changes upon nebulization. EP1941901, WO2020/002650, WO2007/009208, CN1739793, WO2012/021088 disclose varied compositions comprising rhGM-CSF.

### Summary

The present invention discloses a stable liquid formulation of rhGM-CSF, which is ready for use for inhalation and does not experience relevant instability upon nebulization.

In one embodiment of the present invention the combination of a sugar alcohol or a sugar, albumin and polyethylene glycol (PEG) and water provides a highly stable liquid formulation of rhGM-CSF according to claim 1.

In one embodiment the liquid formulation described above is for use as a medicament for nebulization.

In one embodiment the liquid formulation of rhGM-CSF is for use in the treatment of aPAP or NTM infection according to claim 14.

In one embodiment a method for pulmonary delivery of recombinant rhGM-CSF comprising nebulizing rhGM-CSF, a sugar alcohol, albumin and PEG according to claim 1 is disclosed.

In a further embodiment a nebulizer comprising the liquid formulation of rhGM-CSF is disclosed for use in the treatment of aPAP or NTM infection according to claim 14.

### Description of Drawings

**Figure 1****: Impact of Mannitol on % rhGM-CSF remaining after 3 months at 40°C.** The impact of mannitol concentration on stability of liquid formulation of rhGM-CSF was evaluated at accelerated conditions over 3 months. The concentration of mannitol was found to influence drug stability. Higher concentrations (50 mg/mL) of mannitol improved stability of rhGM-CSF. The y-axis is % rhGM-CSF content remaining compared to T0.
**Figure 2****: Impact of rhGM-CSF level within groupings of mannitol conc. on % GM-CSF remaining after 3 months at 40°C**
   The impact of rhGM-CSF concentration on stability of the liquid formulation was also evaluated at accelerated conditions over 3 months. The concentration of rhGM-CSF was found to influence drug stability. Higher concentrations of rhGM-CSF (400 µg/mL) at specific mannitol concentrations improved stability of rhGM-CSF in the liquid formulation. The y-axis is % rhGM-CSF content remaining compared to T0.

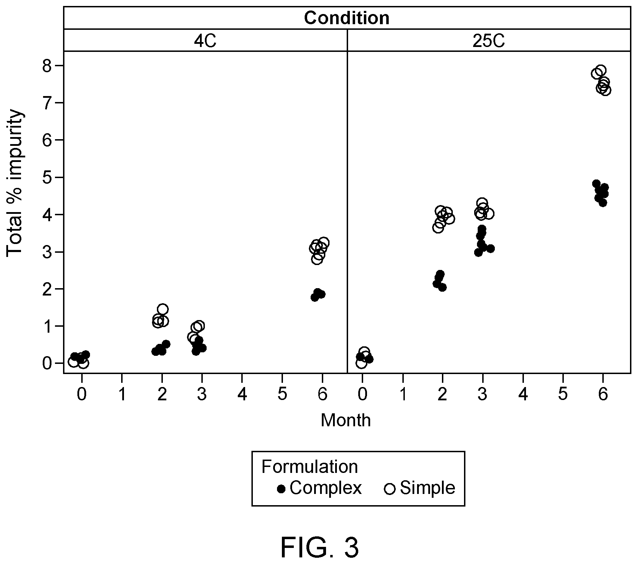

**Figure 3****: Impact of rHA and PEG 4000 on rhGM-CSF impurity formation.**
   Over 6 months at refrigerated and accelerated conditions less rhGM-CSF impurities were formed in a formulation containing rHA and PEG 4000 as a stabilizer. Simple formulation (open circles)contains no rHA or PEG 4000. Complex formulation (solid circles)contains 1.0 mg/mL rHA and 0.1 mg/mL PEG 4000.

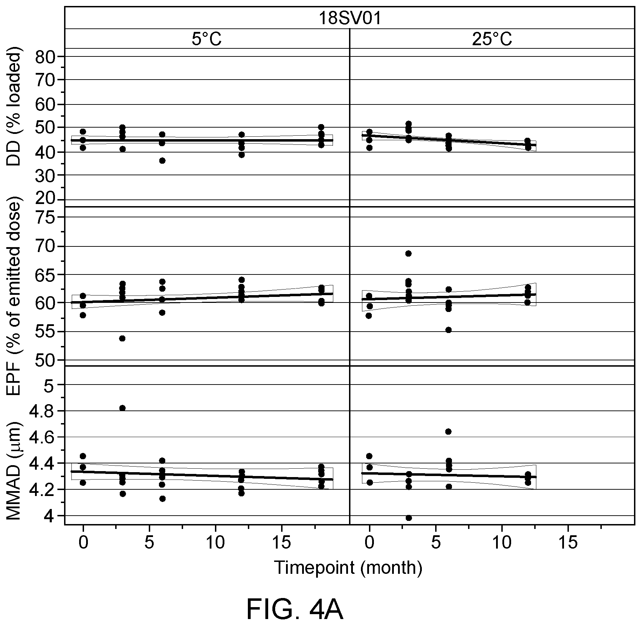

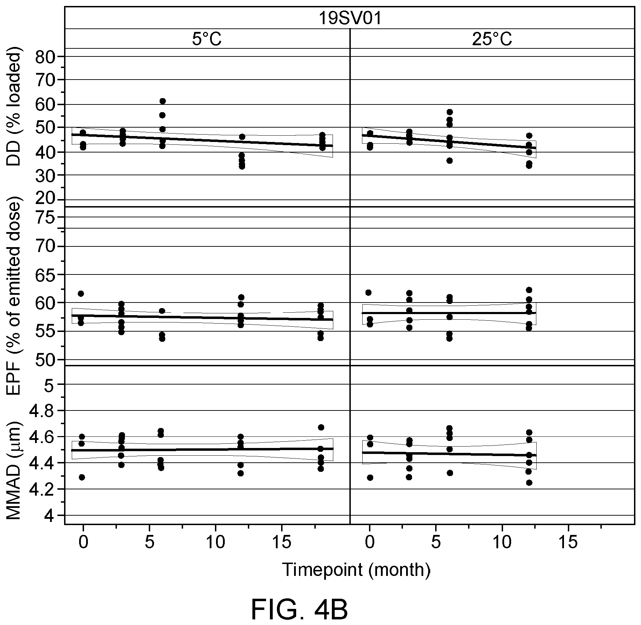

**Figure 4****: 18 Month performance stability of rhGM-CSF complex formulation.** The performance stability of the complex rhGM-CSF formulation upon nebulization was evaluated up to 18 months of storage at 5 and 25°C. The following three parameters were evaluated: DD = Delived dose under simulated breathing (according to USP <1601>, adult), FPF = Fine particle fraction (droplets less than 5 µm) and MMAD = Mass median aerodynamic diameter. Little or no change in performance stability was found upon nebulization at time points up to 18 months. Fig. 4A and 4B represents two separate batches.

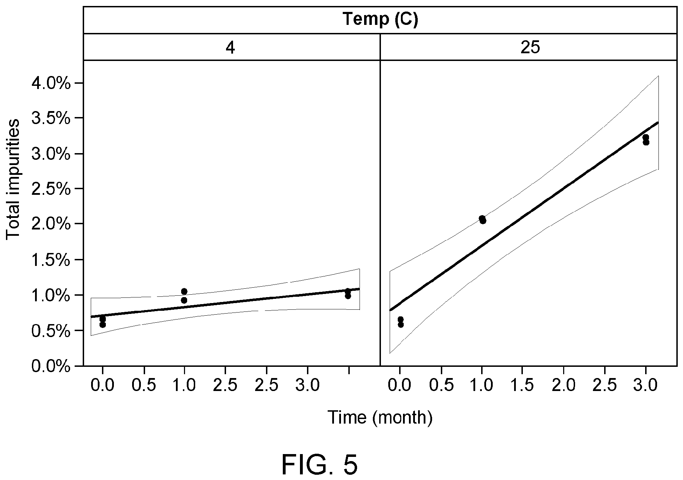

**Figure 5****:** **3** **months stability of low rHA concentration formulation.**
   A version of the complex rhGM-CSF with a low rHA concentration (0.2mg/mL) was evaluated and demonstrated improved stability over the simple formulation that did not include rHA or PEG 4000.

### Detailed description

The present invention relates to a liquid formulation of recombinant human granulocyte macrophage colony stimulating factor (rhGM-CSF)for use in administration by inhalation, wherein the liquid formulation comprises rhGM-CSF, a sugar alcohol or a sugar, albumin and water according to claim 1.

The combination of these specific excipients ensures a high stability of rhGM-CSF in a liquid formulation, which is ready for use for inhalation therapy.

In one embodiment said rhGM-CSF is molgramostim (SEQ ID NO: 1), sargramostim (SEQ ID NO: 2) or regramostim.

The amino acid sequence of molgramostim is identical to the amino acid sequence of the endogenous human GM-CSF. The amino acid sequence of sargramostim differs from natural GM-CSF by a substitution of leucine at position 23. In a preferred embodiment said rhGM-CSF is molgramostim (SEQ ID NO: 1). Molgramostim is an unglycosylated rhGM-CSF expressed in E coli.

In one embodiment the liquid formulation of rhGM-CSF includes a sugar, wherein said sugar is selected from the group consisting of maltose, trehalose, sucrose, mannose, lactose or galactose.

In another embodiment of the present invention the liquid formulation of rhGM-CSF includes a sugar alcohol selected from the group consisting of mannitol Addition of a polyol sugar to the liquid formulation of rhGM-CSF increases the stability. Furthermore, it is used as a tonicity agent to ensure the formulation is isotonic with human plasma. In a preferred embodiment said sugar alcohol is mannitol.

Albumin is a family of globular proteins, the most common of which are the serum albumins. Human serum albumin (HSA) is the most common protein found in human plasma. The addition of HSA contributes to the stability of the rhGM-CSF liquid formulation. In addition, the abundance and natural self-tolerance to albumin minimizes the likelihood of HSA eliciting an immune response in a given patient population. Thus, in one embodiment of the present invention the liquid formulation of rhGM-CSF includes albumin. In a preferred embodiment said albumin is HSA. In another preferred embodiment said albumin is recombinant human albumin (rHA).

In one embodiment of the present invention the liquid formulation of rhGM-CSF further comprises PEG . Such PEGs include, but are not limited to PEG 1000, PEG 1550, PEG 2000, PEG 3000, PEG 3350, PEG 4000 or PEG 8000. Thus, in one embodiment the liquid formulation of rhGM-CSF further comprises a selected from the group consisting of PEG 1000, PEG 1550, PEG 2000, PEG 3000, PEG 3350, PEG 4000 or PEG 8000. In a preferred embodiment and in the invention said PEG is PEG-4000.

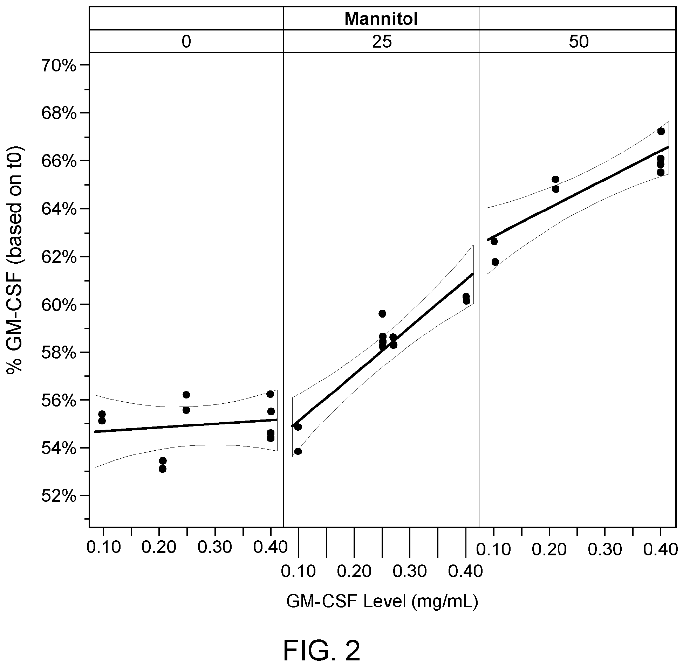

The concentration of protein is crucial to ensure a stable and ready for use formulation. Increasing concentration of rhGM-CSF can increase the stability of the liquid formulation of rhGM-CSF. In one embodiment of the present invention the concentration of rhGM-CSF is at least 150 µg/mL. In a further embodiment of the present invention the concentration of rhGM-CSF in the disclosed liquid formulation is in the range of 150-500 µg/mL. In another embodiment of the present invention the concentration of rhGM-CSF in the disclosed liquid formulation is in the range of 200-300µg/mL. The present disclosure shows that increasing the concentration of rhGM-CSF in the presence of the sugar alcohol mannitol stabilizes the liquid formulation (figure 2).

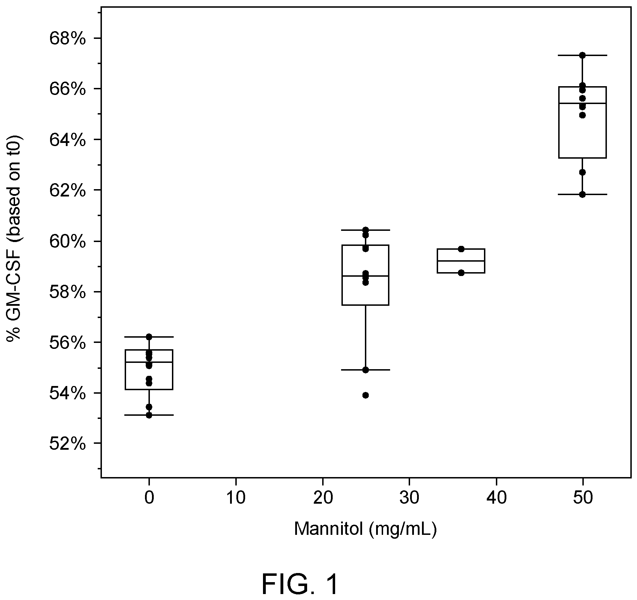

To ensure a balance between isotonicity and stability of the liquid formulation of rhGM-CSF for inhalation an embodiment of the present invention discloses a liquid formulation of rhGM-CSF, wherein the concentration of sugar alcohol or sugar is at least 25 mg/mL. In a further embodiment of the present invention the concentration of sugar alcohol is in the range 45-100 mg/mL. In another embodiment of the present invention the concentration of sugar alcohol is in the range 45-55 mg/mL. As can be seen in figure 1 of the present disclosure increasing the concentration of the sugar alcohol mannitol improves stability of rhGM-CSF. The stabilizing effect of mannitol is observed at a concentration of the sugar alcohol of 25 mg/mL. This effect is significantly increased at a concentration of 50 mg/mL.

In one embodiment of the present invention the liquid formulation of rhGM-CSF comprises albumin in a concentration of at least 0.2 mg/mL. In a further embodiment the liquid formulation of rhGM-CSF comprises albumin in the concentration range 0.2-10 mg/mL, such as 0.3-5 mg/mL such as 0.5-1.5 mg/mL. In figure 3 of the present disclosure it is shown that rhGM-CSF less impurities are formed in a liquid formulation of rhGM-CSF containing rHA and PEG 4000 as stabilizer (complex formulation) in both refrigerated and accelerated conditions. Furthermore, figure 5 displays that a stabilizing effect of albumin can be observed already at concentrations of 0.2 mg/mL, with the stabilizing effect increasing at increasing concentrations.

In one embodiment of the present invention the liquid formulation of rhGM-CSF comprises PEG for example in a concentration of max 50, such as max 40, such as max 30, such as max 20 such as max 10 such as max 1 mg/mL. In another embodiment of the present invention the concentration of PEG is in the concentration range 0.05-0.5 mg/mL such as 0.05-0.3 mg/mL such as 0.05-0.2 mg/mL. In a further embodiment of the present invention the liquid formulation of rhGM-CSF comprises PEG in the concentration range 0.05-0.15 mg/mL.

Selecting the optimal combination and concentration of excipients and biotherapeutic is a highly specific and complex process, which is unique for each individual protein formulation. The present invention discloses one embodiment of the liquid formulation of rhGM-CSF, wherein rhGM-CSF is molgramostim (SEQ ID NO: 1), said sugar alcohol is mannitol, said albumin is recombinant human albumin and said PEG is PEG-4000.

In one embodiment, the concentration of rhGM-CSF is at least 150 µg/mL, the concentration of mannitol is at least 25mg/mL, the concentration of recombinant human albumin is at least 0.2 mg/mL, and the concentration of PEG-4000 is max 1 mg/mL.

In another embodiment, the concentration of rhGM-CSF is 150-500 µg/mL, the concentration of mannitol is 45-100 mg/ml, the concentration of recombinant human albumin is 0.5-1.5 mg/ml, and the concentration of PEG-4000 is 0.05-0.15 mg/mL.

In a preferred embodiment the concentration of rhGM-CSF is in the range of 200-300µg/mL, the concentration of mannitol is 50 mg/ml, the concentration of recombinant human albumin is 1mg/ml and the concentration of PEG-4000 is 0.1 mg/mL.

Buffers are added to a formulation to adjust and stabilize pH and optimize drug solubility and stability. For drugs for inhalation it is desirable that the product pH be close to physiological pH. In one embodiment of the present invention the liquid formulation of rhGM-CSF further comprises a buffer. In a preferred embodiment said buffer comprises citric acid monohydrate and Na₂HPO₄.

The osmolality of the formulation is a key parameter for a liquid formulation to be suitable for inhalation. Preferably, the liquid formulation is essentially isotonic. Thus, in one embodiment of the present invention the osmolality of the formulation is from 250 to 375 mOsm/L, preferably from 325 to 335 mOsm/L.

In one embodiment of the present invention a liquid formulation of rhGM-CSF, wherein the % of rhGM-CSF remaining after 3 months storage at 40°C is more than 60% is disclosed. In a further embodiment of the present invention a liquid formulation of rhGM-CSF, wherein the level of impurity formation after 6 months at 25°C is less than 5% is disclosed.

The present invention further discloses in one embodiment, a method for pulmonary delivery of rhGM-CSF comprising nebulizing rhGM-CSF, a sugar alcohol, albumin and a PEG.

The liquid formulation of rhGM-CSF presented herein is especially well suited for nebulization. Data in the present disclosure demonstrates that the potency of molgramostim for a liquid formulation comprising rhGM-CSF, a sugar alcohol or a sugar, albumin, PEG and water retains 99.4% of the biological potency of rhGM-CSF upon nebulization. Furthermore, it is found that following nebulization of this liquid formulation of rhGM-CSF, rhGM-CSF remains largely intact after the nebulization process. Finally, little or no change in performance stability is found upon nebulization of the liquid formulation presented herein at time points up to 18 months.

In one embodiment of the present invention at least 90% such as at least 95% such as at least 98% such as at least 99% biologic potency of rhGM-CSF is retained after nebulization.

In a further embodiment of the present invention the formulation is storage stable for at least 18 months at 25°C.

In another embodiment of the present invention the liquid formulation is for use in pulmonary administration. To facilitate pulmonary administration a formulation must be aerosolized into small, respirable droplets, where are large fraction are less than 5 µm in diameter. For a biologic preparation, the formulation must maintain stability during the aerosolization process, where high levels of mechanical energy and increased area of air-liquid interface can lead to protein denaturation and/or aggregation. Formulations should also include excipients that are safe for administration to the lungs, and in a suitable range of pH and tonicity.

In another embodiment of the present invention the liquid formulation of rhGM-CSF is for use in the treatment of a lung infection.

In a further embodiment the liquid formulation of rhGM-CSF is for use in the treatment of bacterial, viral or fungal infection in the lungs. In one embodiment the bacterial infection is selected from the group consisting of: Streptococcus pneumoniae, Haemophilus species, Staphylococcus aureus and Mycobacterium tuberculosis. In another embodiment the viral infection is selected from the group consisting of bronchitis, pneumonia, and bronchiolitis. In yet another embodiment the fungal infection is selected from the group consisting of: *Aspergillus, Cryptococcus, Pneumocystis,* and endemic fungi.

In a preferred embodiment the liquid formulation of rhGM-CSF is for use in the treatment of autoimmune pulmonary alveolar proteinosis (aPAP) or non-tuberculous mycobacterial (NTM) infection, tuberculosis, ARDS, influenza infection, coronavirus infection, acute and chronic radiation syndrome, bronchial asthma, COPD, pulmonary fibrosis, lung cancer and respiratory inflammatory disorders. The formulation may also be used prophylactically to stimulate an immunological response and protect against pulmonary infection.

In an embodiment of the present invention, a liquid formulation of rhGM-CSF is disclosed for use as a medicament for nebulization. In another embodiment, a liquid formulation of rhGM-CSF is disclosed for use in the treatment of aPAP or NTM infection.

In one embodiment a method of treating a medical condition comprising administering a liquid formulation of rhGM-CSF by nebulization to a person in need thereof. In another embodiment a method of treating aPAP or NTM infection comprising administering a liquid formulation of rhGM-CSF to a person in need thereof is provided.

APAP is a rare autoimmune lung disorder. It is the most common form (90% of the cases) of pulmonary alveolar proteinosis (PAP). Most cases affect adults between the ages of 20-50 years. Some people may not show symptoms, while others may have progressive difficulty breathing and shortness of breath upon exertion. Other signs and symptoms may include a dry, chronic cough; fatigue; weight loss; chest pain; and a general feeling of ill health. In rare cases, coughing up of blood, rounding and swelling of the tips of the fingers, and cyanosis may be present. Autoimmune PAP is caused by an immune system malfunction, due to IgG antibodies that block the GM-CSF effect. GM-CSF regulates clearance of surfactant (a mix of protein and fat) by alveolar macrophages. The surfactant pile up in the air sacs of the lungs (alveoli), and eventually lead to an inability to breath. The standard treatment is a procedure called lung lavage

NTM are mycobacterial, which do not cause tuberculosis or leprosy (also known as Hansen's disease). NTM do cause pulmonary diseases that resemble tuberculosis. The most common clinical manifestation of NTM disease is lung disease, but lymphatic, skin/soft tissue, and disseminated disease are also important. Pulmonary disease caused by NTM is most often seen in post-menopausal women and patients with underlying lung disease such as cystic fibrosis, bronchiectasis, and prior tuberculosis. It is not uncommon for alpha 1-antitrypsin deficiency, Marfan syndrome and primary ciliary dyskinesia patients to have pulmonary NTM colonization and/or infection. Pulmonary NTM can also be found in individuals with AIDS and malignant disease.

Clinical symptoms vary in scope and intensity but commonly include chronic cough, often with purulent sputum. Hemoptysis may also be present. Systemic symptoms include malaise, fatigue, and weight loss in advanced disease. The diagnosis of M. abscessus pulmonary infection requires the presence of symptoms, radiologic abnormalities, and microbiologic cultures.

An inhaler comprising the liquid formulation of rhGM-CSF according to the present disclosure. In a preferred embodiment the inhaler is a nebulizer. Types of nebulizers include jet nebulizers, ultrasonic and vibrating mesh. Other embodiments include soft-mist inhalers, surface acoustic wave atomization and capillary aerosol generators,

In one embodiment the present invention discloses a method for delivering rhGM-CSF to the lungs comprising nebulization of a liquid formulation of rhGM-CSF, wherein the liquid formulation comprises rhGM-CSF, a sugar alcohol or a sugar, albumin and water; whereby said rhGM-CSF is delivered to the lungs.

In another embodiment the present invention discloses a nebulizer comprising the liquid formulation of rhGM-CSF for use in the treatment of aPAP or NTM infection. This includes jet nebulizers, ultrasonic and vibrating mesh nebulizers.

### Examples

The effect of different formulations on rhGM-CSF stability were studied using HPLC. The content and the purity of each formulation was assessed by Reverse-Phase High Pressure Chromatography (RP-HPLC). The determination of higher order structures (aggregates) and degradation products of rhGM-CSF was assessed by Size Exclusion High Pressure Chromatography (SE-HPLC).

### Example 1 (figure 1) - Effect of mannitol concentration

Three formulation parameters of molgramostim were varied according to the table below (table 1).
1. Mannitol level (0 to 50 mg/mL): buffer conc was also adjusted simultaneously so that the formulation would remain isotonic.
2. Stabilizer (1 of 4 options): rHA and PEG, rHA alone, no stabilizer and tween 80 alone
3. Molgramostim level (0.1 to 0.4 mg/ml)

**Table 1**

| | | Stabilizer | | | | | |
|---|---|---|---|---|---|---|---|
| F # | Mannit ol conc. (mg/ml ) | Recombin ant human albumin (rHA) (mg/mL) | PEG 4000 (mg/m L) | Tween 80 (mg/m L) | Molgramost im conc. (mg/ml) | Dibasic Sodium Phospha te (mg/ml) | Citric Acid Monohydra te (mg/ml) |
| 1 | 50 | 0 | 0 | 0.1 | 0.4 | 2.580 | 0.283 |
| 2 | 25 | 0 | 0 | 0.1 | 0.1 | 7.740 | 0.850 |
| 3 | 25 | 0 | 0 | 0 | 0.2695 | 7.740 | 0.850 |
| 4 | 25 | 1 | 0 | 0 | 0.4 | 7.740 | 0.850 |
| 5 | 0 | 1 | 0.1 | 0 | 0.25 | 15.480 | 1.699 |
| 6 | 0 | 0 | 0 | 0.1 | 0.4 | 15.480 | 1.699 |
| 7 | 25 | 1 | 0 | 0 | 0.25 | 7.740 | 0.850 |
| 8 | 50 | 1 | 0 | 0 | 0.2095 | 2.580 | 0.283 |
| 9 | 50 | 1 | 0.1 | 0 | 0.4 | 2.580 | 0.283 |
| 10 | 25 | 0 | 0 | 0 | 0.25 | 7.740 | 0.850 |
| 11 | 0 | 1 | 0 | 0 | 0.1 | 15.480 | 1.699 |
| 12 | 50 | 0 | 0 | 0 | 0.1 | 2.580 | 0.283 |
| 13 | 0 | 0 | 0 | 0.1 | 0.208 | 15.480 | 1.699 |
| 14 | 0 | 1 | 0.1 | 0 | 0.4 | 15.480 | 1.699 |
| 15 | 33.25 | 1 | 0.1 | 0 | 0.1 | 6.450 | 0.708 |

Data was collected by the RP-HPLC method. The vials were stored for 3 months at 40°C. There were two preparations for each of the 15 formulations in the table above. The primary finding of this study was what is represented figure 1. The level of mannitol had a notable impact on stability at accelerated conditions at 3 months.

### Example 2 (figure 2) - Effect of rhGM-CSF concentration

From the data collected as described in example 1 it was further observed that an increase of the rhGM-CSF concentration also had a notable effect on the stability of the rhGM-CSF formulation at specific concentrations of mannitol.

### Example 3 - Effect of rHA and PEG-4000 (figure 3)

The effect of rHA and PEG-4000 on the stability of molgramostim formulation for nebulization was studied at the following conditions.

**Table 3: Stability Sample Placement for a Single Formulation**

| Storage Conditions | Samples per Stability Time Point | | | | | | Reserve | Total Quantity Required per Condition |
|---|---|---|---|---|---|---|---|---|
| | Initial T=0 | 0.5 Month | 1 Months | 3 Months | 6 Months | Optional | | |
| 4°C/Ambient RH | 9 vials | | 12 vials | 12 vials | 18 vials | 18 vials | 12 vials | 81 vials |
| 25°C/60% RH | | | 12 vials | 12 vials | 18 vials | 18 vials | 12 vials | 72 vials |
| 40°C/75% RH | | 12 vials | 12 vials | 12 vials | | 18 vials | 12 vials | 66 vials |
| Total | 129 vials | | | | | 54 vials | 36 vials | 219 vials |

### Formulation preparation

The stability studies were performed using versions (complex and simple) of molgramostim formulation, as described in the following.

### Molgramostim Complex Formulation

The complex formulation was prepared on a weight basis according to the description below. Sterile filtration was only done on the final formulated product. The content of the final formulated product is according to the table below.

| **Ingredient** | **Amount per Vial (1.2 mL)** |
|---|---|
| Molgramostim | 0.300 mg |
| Mannitol | 60 mg |
| Citric acid monohydrate | 0.34 mg |
| Disodium hydrogen phosphate, anhydrous | 3.1 mg |
| Recombumin Prime (rHSA) | 1.2 mg |
| PEG 4000 | 0.120 mg |
| Water for Injection | to 1.2 mL |

For manufacturing of the complex rhGM-CSF formulation the buffer was prepared first according to the following procedure:

### Preparation of Citrate/Phosphate/HSA Buffer (Example Batch Size = 2000 q)

| **Ingredient** | **Amount for 2000 g batch** |
|---|---|
| Mannitol | 94.87±0.9 g |
| PEG 4000 | 0.190 ±0.002 g |
| Citric acid monohydrate | 0.531 ± 0.05 g |
| Disodium hydrogen phosphate, anhydrous | 4.934 ± 0.49 g |
| Recombumin Prime (rHSA) | 9.488 ± 0.09 g |
| Water for Injection | To 2000.0 ± 1.0 g |

The rHSA was removed from 2-8°C storage and allow to equilibrate to room temperature. The required amounts of mannitol, PEG 4000, citric acid monohydrate, disodium hydrogen phosphate (anhydrous), and rHSA were pre-weighed in appropriate containers. A clean beaker/bottle and stir bar was placed on a top loading balance and the weight of the beaker and stir bar was recorded and tared. Approximately 800 g of Water for Injection (WFI) was added to the beaker. The required amounts of citric acid monohydrate, disodium hydrogen phosphate (anhydrous), mannitol, and PEG 4000 was added to the WFI and the beaker was transferred to a stir plate and the mixture was stirred until all solids were dissolved. The beaker was placed back on the top loading balance and the required amount of rHSA was added to the beaker. Then the beaker was transferred to a stir plate and the solution was gently stirred for approximately 5 min or until the solution is homogenous. Foaming should be avoided. Finally, WFI was added to a target amount of 2000 g and the solution was again stirred gently for approximately 10 min while foaming was avoided. The buffer was labeled and stored at 2-8°C until further processed. This buffer can be stored at 2-8 °C for up to 100 hours.

### Preparation of rhGM-CSF Formulated Product (Example Batch Size = 1000 g)

Following the preparation of the buffer the complex rhGM-CSF formulation is prepared according to the following procedure:
Weight (g) of rhGM-CSF solution = A
Weight (g) of formulated product = A + (10.1507 · A)

| **Ingredient** | **Amount for 1000 g batch** |
|---|---|
| rhGM-CSF (2.74 mg/mL) | 100 g |
| Citrate Phosphate Buffer with HSA | To 1115.71 ± 1.11 g |

The rhGM-CSF was thawed for 24h ± 4h at 2-8°C. If the bulk material is not thawed within this timeframe, thawing is continued at room temperature by gentle turning of the bottle. The bottle should not be shaken and foaming should be avoided. The required amount of rhGM-CSF was pre-weighed into an appropriately sized container. A clean bottle and stir bar were placed on a top loading balance. The weight of the beaker and stir bar was recorded and tared. Approximately 800 g of Citrate/Phosphate/rHA Buffer was added to the beaker and then the pre-weighed amount of rhGM-CSF was also added to the beaker. The rhGM-CSF container was rinsed with Citrate/Phosphate/HSA Buffer and the rinse was transferred to the beaker. The beaker was then placed on a stir plate and the mixture was gently stirred for approximately 5 min while avoiding foaming. The mixture was checked for complete homogeneity. Citrate/Phosphate/HSA Buffer was added to reach a final weight of 1000 g followed by gently stirring for approximately 10 min while avoiding foaming. The formulated product was filtered using Millipore Stericup filter units (PVDF membrane (Durapore), very low protein binding, pore size 0.22 µm) and the formulated product was stored at 2-8°C, protected from light.

### Molgramostim Simple Formulation

The simple rhGM-CSF formulation is prepared on a volumetric basis because the density of the simple formulation buffer is unknown. The concentrations and amounts per vial of the ingredients in the final formulated product is shown in the table below.

| **Ingredient** | **Concentratlon (mg/mL)** | **Amount per Vial (1.2 mL)** |
|---|---|---|
| Molgramostim | 0.250 | 0.300 mg |
| Mannitol | 50.0 | 60 mg |
| Citric acid monohydrate | 0.283 | 0.34 mg |
| Disodium hydrogen phosphate, anhydrous | 2.58 | 3.1 mg |
| Water for Injection | To 1 mL | To 1.2 mL |

### Preparation of rhGM-CSF Formulated Product (Example Batch Size = 1000 mL)

| **Ingredient** | **Amount for 1000 g batch** |
|---|---|
| rhGM-CSF (2.74 mg/mL, density = 1 g/mL)) | 91.24 ± 0.10 g |
| Mannitol | 50.0 ± 0.5 g |
| Citric acid monohydrate | 0.283 ± 0.003 g |
| Disodium hydrogen phosphate, anhydrous | 2.58 ± 0.03 g |
| Water for Injection | To 1000.0 g ± 1.0 g |

The required amounts of mannitol, citric acid monohydrate, disodium hydrogen phosphate (anhydrous) was pre-weighed in appropriate containers. Approximately 800 mL of Water for Injection (WFI) was added to a beaker with a stir bar. Citric acid monohydrate, disodium hydrogen phosphate (anhydrous), and mannitol was added to the WFI. The mixture was stirred until all solids were dissolved. The required amount of rhGM-CSF was added to the beaker and the rhGM-CSF container was rinsed with WFI and the rinse was transferred to the beaker. The beaker was placed on a stir plate and the mixture was gently stirred for approximately 5 min while foaming was avoided. Check for complete homogeneity.

The contents of the beaker was transferred to a 1L volumetric flask. The beaker was rinsed with WFI and the rinse was transferred to the volumetric flask. Care should be taken not to generate foam during the transfer. WFI was added to the volumetric flask to QS to 1L and the contents were mixed by gentle inversion of the flask. The formulated product was filtered using Millipore Stericup filter units (PVDF membrane (Durapore), very low protein binding, pore size 0.22 µm). The final formulated product is stored at 2-8°C, protected from light.

The main finding of this study was what is represented figure 2. Less rhGM-CSF impurities were formed in a formulation containing rHA and PEG 4000 as a stabilizer (complex formulation) in both refrigerated and accelerated conditions. Furthermore, the tables below show that the complex formulation has a clearly higher purity upon storage for 12 days at 40°C compared to the simple formulation:

### Simple formulation 40 °C

| **packaging material** | **purity, mean value** | **content [mg/mL], mean value** |
|---|---|---|
| 6R glass vial | 88,0% | 0.30 |
| PP syringe | 92,0% | 0,30 |
| glass syringe | 91,3% | 0,30 |

### Complex formulation 40 °C

| **packaging material** | **purity, mean value** | **content [mg/mL], mean value** |
|---|---|---|
| 6R glass vial | 94,7% | 0,31 |
| PP syringe | 94,9% | 0,30 |
| glass syringe | 94,9% | 0,30 |

### Example 4 - Stability upon nebulization

The stability of the rhGM-CSF complex formulation following nebulization was evaluated by comparing un-nebulized formulation with condensed nebulized formulation and comparing detected impurities and protein aggregates.

Aerosol generated by a vibrating mesh was collected directly after nebulization by collecting in a polypropylene tube positioned against the mesh.

Impurities and protein aggregates were detected by High Performance Liquid Chromatography (HPLC) and Size Exclusion Chromatography (SEC), respectively. The formulation evaluated in this study was identical to the rhGM-CSF complex formulation described above except that the rhGM-CSF concentration was 300 µg/mL rather than 250 µg/mL. A high concentration of molgramostim might be more likely to demonstrate instability, representing a worst-case scenario.

Detection of unknown impurities determined by HPLC are listed in table 2 below. The relative retention time (RRT) of these peaks to the primary peak were 0.87, 0.96 and 1.07. After nebulization using a Head type 30 device, a slight elevation of RRT 0.87 was detected in the nebulized sample (0.26%) and residue in the reservoir (0.32%). These results demonstrate that rhGM-CSF remains largely intact after the nebulization process.

**Table 2: Comparison of Related Impurities by RP-HPLC**

| | # | RTT 0.87 (% Area) | RTT 0.96 (% Area) | RTT 1.07 (% Area) |
|---|---|---|---|---|
| Untreated Sample | 1 | 0.22 | 0.48 | 0.49 |
| | 2 | No peak | 0.64 | 0.40 |
| Nebulized Sample | 1 | 0.26 | 0.45 | 0.40 |
| | 2 | 0.26 | 0.45 | 0.27 |
| | 3 | 0.27 | 0.40 | 0.36 |
| Sample Remaining in Reservoir | 1 | 0.30 | 0.28 | 0.36 |
| | 2 | 0.36 | 0.56 | 0.44 |
| | 3 | 0.31 | 0.50 | 0.60 |

Initial investigation of impurity and aggregate generation due to nebulization was evaluated by capturing and analyzing aerosol produced by a vibrating mesh nebulizer (PARI eFlow). Upon evaluation of aggregation by SEC, an observable difference was noted in albumin-associated aggregates, denoted as HMW 1 and HMW 2. This data infers that some aggregation of albumin is caused by the nebulization process, however, the majority of the protein in the formulation remains non-aggregated.

**Table 3: Comparison of High Molecular Weight (HMW) Impurities by SE-UPLC**

| | HMW 1 (% Total Area) | HMW 2 (% Total Area) | rHA (% Total Area) | rhGM-CSF (% Total Area) | LMW (% Total Area) | Unk (% Total Area) |
|---|---|---|---|---|---|---|
| Untreated Sample | -- | 1.89 | 69.53 | 26.55 | 2.03 | -- |
| Nebulized Sample | 0.69 | 8.73 | 62.62 | 25.71 | 2.02 | -- |
| Sample Remaining in Reservoir | -- | 2.01 | 68.65 | 25.80 | 2.10 | 0.48 |

**Table 4: Molgramostim content before and after nebulization**

| **Nebulizer Head Type** | **Sample** | **Mean Molgramostim Content (µg/mL) ± S.D. (n=3)** | **Content Remaining after Nebulization (%)** |
|---|---|---|---|
| Head Type 30 | Non-Nebulized | 297.9 ± 5.1 | -- |
| | Nebulized | 282.5 ± 2.7 | 94.8 |
| Head Type 40 | Non-Nebulized | 290.8 ± 0.4 | -- |
| | Nebulized | 277.9 ± 0.6 | 95.6 |

Data collected in Table 4 is the rhGM-CSF concentration of complex formulation before and after nebulization by a vibrating mesh nebulizer. Content was measured by RP-HPLC.

Finally, the potency of molgramostim was also evaluated before and after nebulization and it was found that upon nebulization the 99.4% of the biological potency of GM-CSF was retained for the complex formulation. Biological potency was determined by TF-1 cell line assay

### Example 5 - Performance stability of rhGM-CSF complex formulation.

The performance stability of the complex rhGM-CSF formulation upon nebulization was evaluated up to 18 months of storage at 5 and 25°C. The following three parameters were evaluated: DD = Delived dose under simulated breathing (according to USP <1601>, adult), FPF = Fine particle fraction (droplets less than 5 µm) and MMAD = Mass median aerodynamic diameter. Little or no change in performance stability was found upon nebulization at time points up to 18 months. Performance data were generatred by pharmaceutical cascade impaction according to USP <601>.

### Example 6 - Stability of low rHA concentration formulation

A version of the complex rhGM-CSF with a low rHA concentration (0.2mg/mL) was evaluated and demonstrated improved stability over the simple formulation that did not include rHA or PEG 4000. rhGM-CSF content was measured by RP-HPLC.

## Claims

1. A stable liquid formulation of recombinant human granulocyte macrophage colony stimulating factor (rhGM-CSF), comprising:
a. rhGM-CSF at a concentration of at least 150 ug/mL;
b. mannitol at a concentration of at least 25 mg/mL;
c. recombinant human albumin (rHA) at a concentration of at least 0.2 mg/mL; and
d. polyethylene glycol-4000 (PEG-4000) at a concentration of at most 1 mg/mL.

2. The stable liquid formulation of claim 1, wherein:
the concentration of the rhGM-CSF is from 150 µg/mL to 500 µg/mL;
the concentration of the rHA is from 0.5 mg/mL to 1.50 mg/mL;
the concentration of the PEG-4000 is from 0.05 mg/mL to 0.15 mg/mL; and
the concentration of the mannitol is from 45 mg/mL to 100 mg/mL.

3. The stable liquid formulation of claim 1, wherein:
the concentration of the rhGM-CSF is from 200 µg/mL to 300 µg/mL;
the concentration of the recombinant human albumin is from 0.5 mg/mL to 1.50 mg/mL;
the concentration of the PEG-4000 is from 0.05 mg/mL to 0.15 mg/mL; and
and the concentration of the mannitol is from 45 mg/mL to 100 mg/mL.

4. The stable liquid formulation of claim 1, wherein the rhGM-CSF is molgramostim (SEQ ID NO: 1), sargramostim (SEQ ID NO: 2), or regramostim.

5. The stable liquid formulation of claim 1, wherein less than 5% of impurity is formed in the stable liquid formulation after 6 months of storage at 25 °C.

6. The stable liquid formulation of claim 5, wherein the impurity is determined using HPLC.

7. The stable liquid formulation according to any one of the preceding claims, further comprising a buffer.

8. The stable liquid formulation of claim 7, wherein the buffer is citric acid monohydrate or Na₂HPO₄.

9. The stable liquid formulation according to any one of the preceding claims, wherein the osmolality of the formulation is from 250 to 375 mOsm/L, preferably from 325 to 335 mOsm/L.

10. The stable liquid formulation of any one of claims 1-9, wherein the stable liquid formulation is nebulized.

11. The stable liquid formulation of claim 10, wherein at least 90% of a biologic potency of rhGM-CSF is retained after nebulization.

12. An inhaler comprising the stable liquid formulation of any one of claims 1-11, preferably wherein the inhaler is a nebulizer.

13. The stable liquid formulation of any one of claims 1-11 formulated for pulmonary administration.

14. The stable liquid formulation of any one of claims 1-11 for use in treatment of lung infection; bacterial, viral, or fungal infection in the lungs; or autoimmune pulmonary alveolar proteinosis (aPAP) or non-tuberculous mycobacterial (NTM) infection.

15. The stable liquid formulation of any one of claims 1-11 for use in delivering rhGM-CSF to the lungs.

## Patentansprüche

1. Stabile flüssige Formulierung von rekombinantem humanem Granulozyten-Makrophagen-Kolonie-stimulierendem Faktor (rhGM-CSF), umfassend:
a. rhGM-CSF in einer Konzentration von mindestens 150 µg/ml;
b. Mannitol in einer Konzentration von mindestens 25 mg/ml;
c. rekombinantes humanes Albumin (rHA) in einer Konzentration von mindestens 0,2 mg/ml; und
d. Polyethylenglykol-4000 (PEG-4000) in einer Konzentration von höchstens 1 mg/ml.

2. Stabile flüssige Formulierung nach Anspruch 1, wobei:
die Konzentration des rhGM-CSF zwischen 150 µg/ml und 500 µg/ml ist;
die Konzentration des rHA zwischen 0,5 mg/ml und 1,50 mg/ml ist;
die Konzentration des PEG-4000 zwischen 0,05 mg/ml und 0,15 mg/ml ist; und
die Konzentration des Mannitol zwischen 45 mg/ml und 100 mg/ml ist.

3. Stabile flüssige Formulierung nach Anspruch 1, wobei:
die Konzentration des rhGM-CSF zwischen 200 µg/ml und 300 µg/ml ist;
die Konzentration des rekombinanten humanen Albumins zwischen 0,5 mg/ml und 1,50 mg/ml ist;
die Konzentration des PEG-4000 zwischen 0,05 mg/ml und 0,15 mg/ml ist; und
und die Konzentration des Mannitol zwischen 45 mg/ml und 100 mg/ml ist.

4. Stabile flüssige Formulierung nach Anspruch 1, wobei der rhGM-CSF Molgramostim (SEQ ID NO: 1), Sargramostim (SEQ ID NO: 2) oder Regramostim ist.

5. Stabile flüssige Formulierung nach Anspruch 1, wobei sich nach 6 Monaten Lagerung bei 25 °C weniger als 5 % Verunreinigung in der stabilen flüssigen Formulierung bilden.

6. Stabile flüssige Formulierung nach Anspruch 5, wobei die Verunreinigung mithilfe von HPLC bestimmt wird.

7. Stabile flüssige Formulierung nach einem der vorhergehenden Ansprüche, ferner umfassend einen Puffer.

8. Stabile flüssige Formulierung nach Anspruch 7, wobei der Puffer Citronensäuremonohydrat oder Na₂HPO₄ ist.

9. Stabile flüssige Formulierung nach einem der vorhergehenden Ansprüche, wobei die Osmolalität der Formulierung zwischen 250 und 375 mOsm/l, vorzugsweise zwischen 325 und 335 mOsm/l, ist.

10. Stabile flüssige Formulierung nach einem der Ansprüche 1 bis 9, wobei die stabile flüssige Formulierung vernebelt ist.

11. Stabile flüssige Formulierung nach Anspruch 10, wobei mindestens 90 % der biologischen Wirksamkeit von rhGM-CSF nach der Vernebelung erhalten sind.

12. Inhalator, umfassend die stabile flüssige Formulierung nach einem der Ansprüche 1 bis 11, wobei der Inhalator vorzugsweise ein Vernebler ist.

13. Stabile flüssige Formulierung nach einem der Ansprüche 1 bis 11, formuliert zur pulmonalen Verabreichung.

14. Stabile flüssige Formulierung nach einem der Ansprüche 1 bis 11 zur Verwendung bei der Behandlung von Lungeninfektionen; bakteriellen, viralen oder Pilzinfektionen in der Lunge; oder autoimmuner pulmonaler Alveolarproteinose (aPAP) oder nichttuberkulöser Mykobakterieninfektion (NTM).

15. Stabile flüssige Formulierung nach einem der Ansprüche 1 bis 11 zur Verwendung beim Darreichen von rhGM-CSF in die Lunge.

## Revendications

1. Formulation liquide stable de facteur de stimulation des colonies de granulocytes et macrophages humain recombinant (rhGM-CSF), comprenant :
a. un rhGM-CSF à une concentration d'au moins 150 µg/ml ;
b. du mannitol à une concentration d'au moins 25 mg/ml ;
c. de l'albumine humaine recombinante (rHA) à une concentration d'au moins 0,2 mg/ml ; et
d. du polyéthylène glycol-4000 (PEG-4000) à une concentration d'au plus 1 mg/ml.

2. Formulation liquide stable selon la revendication 1, dans laquelle :
la concentration du rhGM-CSF est de 150 µg/ml à 500 µg/ml ;
la concentration du rHA est de 0,5 mg/ml à 1,50 mg/ml ;
la concentration du PEG-4000 est de 0,05 mg/ml à 0,15 mg/ml ; et
la concentration du mannitol est de 45 mg/ml à 100 mg/ml.

3. Formulation liquide stable selon la revendication 1, dans laquelle :
la concentration du rhGM-CSF est de 200 µg/ml à 300 µg/ml ;
la concentration de l'albumine humaine recombinante est de 0,5 mg/ml à 1,50 mg/ml ;
la concentration du PEG-4000 est de 0,05 mg/ml à 0,15 mg/ml ; et
la concentration du mannitol est de 45 mg/ml à 100 mg/ml.

4. Formulation liquide stable selon la revendication 1, dans laquelle le rhGM-CSF est le molgramostim (SEQ ID N° : 1), le sargramostim (SEQ ID N° : 2) ou le regramostim.

5. Formulation liquide stable selon la revendication 1, dans laquelle moins de 5 % d'impuretés se forment dans la formulation liquide stable après 6 mois de stockage à 25 °C.

6. Formulation liquide stable selon la revendication 5, dans laquelle l'impureté est déterminée par HPLC.

7. Formulation liquide stable selon l'une quelconque des revendications précédentes, comprenant en outre un tampon.

8. Formulation liquide stable selon la revendication 7, dans laquelle le tampon est l'acide citrique monohydraté ou Na₂HPO₄.

9. Formulation liquide stable selon l'une quelconque des revendications précédentes, dans laquelle l'osmolalité de la formulation est de 250 à 375 mOsm/l, de préférence de 325 à 335 mOsm/l.

10. Formulation liquide stable selon l'une quelconque des revendications 1 à 9, dans laquelle la formulation liquide stable est nébulisée.

11. Formulation liquide stable selon la revendication 10, dans laquelle au moins 90% de la puissance biologique de rhGM-CSF est conservée après nébulisation.

12. Inhalateur comprenant la formulation liquide stable selon l'une quelconque des revendications 1 à 11, de préférence dans lequel l'inhalateur est un nébuliseur.

13. Formulation liquide stable selon l'une quelconque des revendications 1 à 11, formulée pour administration pulmonaire.

14. Formulation liquide stable selon l'une quelconque des revendications 1 à 11, destinée à être utilisée dans le traitement d'infections pulmonaires ; d'infections bactériennes, virales ou fongiques des poumons ; ou de la protéinose alvéolaire pulmonaire auto-immune (aPAP) ou d'une infection par mycobactéries non tuberculeuses (MNT).

15. Formulation liquide stable selon l'une quelconque des revendications 1 à 11, destinée à être utilisée dans l'administration de rhGM-CSF aux poumons.
